# EUROPÄISCHE PATENTSCHRIFT

(11) **EP 0 705 102 B1**
(45) Veröffentlichungstag und Bekanntmachung des Hinweises auf die Patenterteilung: **20.08.1997**
(21) Anmeldenummer: 94918874.2
(22) Anmeldetag: 15.06.1994
(51) Int. Cl.: A61K 31/68

(54) **VERWENDUNG VON CORRINOIDEN ZUR TOPISCHEN ANWENDUNG BEI HAUTERKRANKUNGEN**
USE OF CORRINOIDS IN TOPICAL TREATMENT OF SKIN DISEASES
UTILISATION DES CORRINOIDES EN APPLICATION LOCALE POUR LES AFFECTIONS CUTANEES

(30) Priorität: 15.06.1993 DE 4319629
(43) Veröffentlichungstag der Anmeldung: 10.04.1996
(73) Patentinhaber: KLINGELHOLLER, Karsten, D-42275 Wuppertal (DE)
(72) Erfinder: KLINGELHOLLER, Karsten, D-42275 Wuppertal (DE)
(74) Vertreter: Christophersen, Ruth
(86) Internationale Anmeldenummer: EP9401951
(87) Internationale Veröffentlichungsnummer: WO9428907

(56) Entgegenhaltungen:
- EP-A- 0 055 118
- WO-A-94/13252
- DE-A- 1 922 192
- DE-A- 2 325 809
- DE-A- 2 505 114
- DE-A- 3 210 669
- US-A- 3 548 057
- US-A- 3 577 537
- J.NUTR., Bd.92, Nr.2, Juni 1967 Seiten 261 - 266 E.E.HOWE ET AL. 'Percutaneous Absorption of Vitamin B12 in the Rat and Guinea Pig'
- 'Vitamin Compendium' 1976 , VITAMINS AND CHEMICALS DEPARTMENT, F.HOFFMANN-LA ROCHE & CO. LTD. , BASLE, SWITZERLAND siehe Seite 110 - Seite 115
- CHEMICAL ABSTRACTS, vol. 88, no. 11, 13. März 1978, Columbus, Ohio, US; abstract no. 72417f, R.G.GADZHIEV ET AL. 'Status of blood electrolytes in patients with allergic dermatitis'

## Beschreibung

Die Erfindung betrifft eine neue Verwendung von Corrinoiden zur Herstellung eines Medikaments zur topischen Behandlung von Hauterkrankungen, insbesondere von entzündlichen und hyperproliferativen Hauterkrankungen bzw. kutanen Manifestationen immunologisch bedingter Erkrankungen, z.B. von: Psoriasis, atopischer Dermatitis, Kontakt-Dermatitis und anderen ekzematösen Dermatitiden, seborrhoeischer Dermatitis, Neurodermitis, Decubitus, Lichen planus, Pemphigus, bullosem Pemphigoid, Epidermolysis bullosa, Urticaria, Angioödemen, Vasculitiden, Erythemen, kutanen Eosinophilien, Lupus erythematodes sowie von Alopecia areata und Haarwachstumsstörungen.

Die Behandlung von Hauterkrankungen, insbesondere von chronischen Hauterkrankungen, stellt in der Medizin ein großes Problem dar, da sie nur sehr begrenzt heilbar sind. Die Behandlung dieser Erkrankungen verschafft in vielen Fällen den Patienten nur eine geringe Linderung, in vielen Fällen ist gar kein Heilungserfolg zu beobachten. Hinzukommt, daß eine Vielzahl der eingesetzten Wirkstoffe wie z. B. Kortison starke Nebenwirkungen aufweisen.

Aus dem Stand der Technik sind Mittel bekannt, die u. a. Vitamin B₁₂ als einen Bestandteil enthalten. So wird beispielsweise in der EP-A-0 055 118 ein Mittel beschrieben, das Vitamin B₁₂ enthält und zur Behandlung von Arthritis und Psoriasis eingesetzt wird.

DE-A-3 210 669 offenbart ein pharmazeutisches Mittel zur Behandlung von Hautkrankheiten, Schleimhauterkrankungen und Nagelwuchsstörungen. Das Mittel besteht im wesentlichen aus Orotsäure oder einem Salz davon und Inosin. Als weiteren Bestandteil kann das Mittel Vitamin B₁₂ enthalten.

Diese Druckschriften befassen sich jedoch nicht mit der topischen Behandlung von chronischen Hauterkrankungen.

Aufgabe der vorliegenden Erfindung ist es, einen wirksamen Wirkstoff zur Behandlung von Hauterkrankungen, insbesondere der oben aufgezählten zur Verfügung zu stellen, der möglichst keine Nebenwirkungen zeigt.

Überraschenderweise wurde nur gefunden, daß Verbindungen mit der Formel I 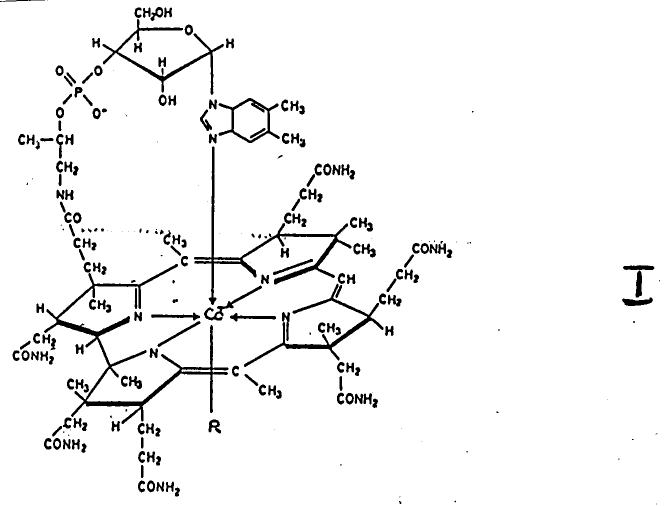 in der
R für CN, OH, CH₃ oder H₂O stehen,
in Verbindung mit einem synthetischen oder pflanzlichen Öl oder Fett zur Herstellung eines Medikaments zur topischen Behandlung von von entzündlichen und hyperproliferativen Hauterkrankungen bzw. kutanen Manifestationen immunologisch bedingter Erkrankungen,
z.B. von: Psoriasis, atopischer Dermatitis, Kontakt-Dermatitis und anderen ekzematösen Dermatitiden, seborrhöischer Dermatitis, Neurodermitis, Decubitus, Lichen planus, Pemphigus, bullosem Pemphigoid, Epidermolysis bullosa, Urticaria, Angioödemen, Vasculitiden, Erythemen, kutanen Eosinophilien, Lupus erythematodes sowie von Alopecia areata und Haarwachstumsstörungen eine hervorragende Wirkung zeigen.

Die Verbindungen mit der Formel I sind auch unter der Bezeichnung Cobalamine oder Vitamin B₁₂ bekannt. Sie werden in der Medizin z. B. zur Behandlung von perniziöser Anämie oral oder subkutan in Form von Tabletten bzw. als Injektionslösung verabreicht.

Für die obige Verwendung hängt die zu verabreichende Dosis von der verwendeten Verbindung, der Verabreichungsart sowie der Behandlungsart ab. Es werden sehr gute Ergebnisse erhalten, wenn eine oder mehrere Verbindungen mit der Formel I mit einer Konzentration von 1 x 10⁻⁴ bis 1 x 10⁻² Gew.-% einmal oder mehrmals täglich lokal verabreicht werden. Die Darreichungsformen können die in der pharmazeutischen Industrie üblichen Träger- bzw. Verdünnungstoffe enthalten. Beispiele von geeigneten galenischen Formen sind Lösungen, Emulsionen, Suspensionen, Lotionen, Gele, Salben oder Cremes.

Die Verbindungen mit der Formel I werden in Kombination mit einem synthetischen oder natürlich vorkommendem Öl oder Fett appliziert. Als besonders geeignet hat sich die Verwendung von pflanzlichen Ölen, insbesondere von Mandelöl, Erdnußöl, Sesamöl, Olivenöl, Weizenkeimöl, Maiskeimöl, Diestelöl, Sojaöl, Sonnenblumenöl, Kokosfett, Avocadoöl, Palmkernöl und Kakaobutter erwiesen.

Die Wirkungen der Verbindungen mit der Formel I ist aus den folgenden Tests ersichtlich:

### Beispiele

Herstellung einer Salbe: 350 ml Avocadoöl, 350 ml dest. Wasser, 70 ml D-Panthenol, 525 mg Cyanocobalamin, 175mg Hydroxycobalamin und 100 g Emulgator, z. B. erhältlich unter den Handelsnamen Euxyl K, Lamecreme oder Emulsan, vermischt und anschließend auf 1000 ml aufgefüllt.

### Beispiel 1

### Wirkung bei Psoriasis

Bei einer Gruppe von 10 Probanden, die unter Psoriasis leiden, wurde die oben hergestellte Salbe 3 mal täglich auf die erkrankten Hautstellen aufgetragen. Die Wirkung der erfindungsgemäßen Salbe auf die erkrankte Haut wurde für eine Hautfläche von 10 cm² beobachtet.

| Applikation/Tage | erkrankte Hautfläche/cm² |
|---|---|
| 1 | 10 |
| 5 | 9 |
| 10 | 8 |
| 20 | 4 |
| 25 | 2 |
| 30 | 0 |

### Beispiel 2

### Wirkung bei Neurodermitis

Wie in Beispiel 1 wurde bei einer Gruppe von 10 Probanden, die unter Neurodermitis leiden, die oben hergestellte Salbe 3 mal täglich auf die erkrankten Hautstellen aufgetragen. Die Wirkung der erfindungsgemäßen Salbe auf die erkrankte Haut wurde für eine Hautfläche von 10 cm² beobachtet.

| Applikation/Tage | erkrankte Hautfläche/cm² |
|---|---|
| 1 | 10 |
| 5 | 8 |
| 10 | 6 |
| 20 | 4 |
| 25 | 2 |
| 30 | 0 |

Aus den obigen Beispielen ist ersichtlich, daß die erfindungsgemäße Verwendung von Verbindungen mit der Formel I nicht nur lindert sondern die erkrankten Hautflächen in einem relativ kurzem Zeitraum auf eine Fläche bis zu 0 cm² vermindert.

## Patentansprüche

1. Verwendung von Verbindungen mit der Formel 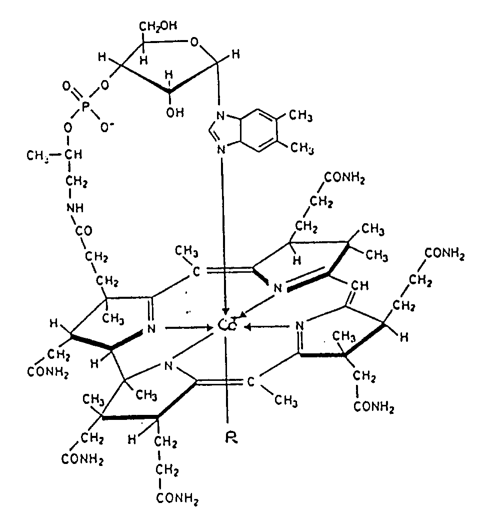 in der
R für CN, OH, CH₃ oder H₂O stehen,
in Verbindung mit einem synthetischen oder pflanzlichen Öl oder Fett zur Herstellung eines Medikaments zur topischen Behandlung von von entzündlichen und hyperproliferativen Hauterkrankungen bzw. kutanen Manifestationen immunologisch bedingter Erkrankungen.

2. Verwendung nach Anspruch 1, dadurch gekennzeichnet, daß es bei den entzündlichen und hyperproliferativen Hauterkrankungen bzw. kutanen Manifestationen immunologisch bedingter Erkrankungen um Psoriasis, atopischer Dermatitis, Kontakt-Dermatitis und anderen ekzematösen Dermatitiden, seborrhoeischer Dermatitis, Neurodermitis, Decubitus, Lichen planus, Pemphigus, bullosem Pemphigoid, Epidermolysis bullosa, Urticaria, Angioödemen, Vasculitiden, Erythemen, kutanen Eosinophilien und Lupus erythematodes handelt.

3. Verwendung nach Anspruch 1 oder 2, dadurch gekennzeichnet, daß übliche Träger- und/oder Verdünnungstoffe verwendet werden.

## Claims

1. Use of compounds characterized by the Formula I 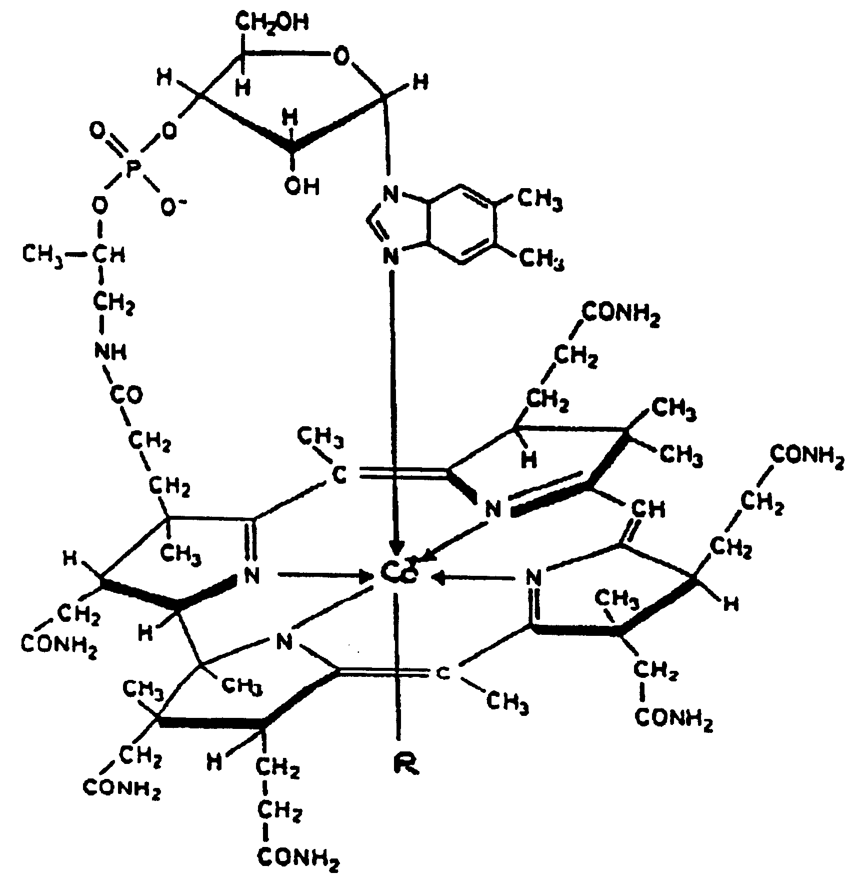 in which
R stands for CN, OH, CH₃ or H₂O,
in combination with a synthetic or vegetable oil or fat for production of a medicament for the topical treatment for inflammatory and hyperproliferative dermatological illnesses, respectively cutaneous manifestations of illnesses which are immunologic in origin.

2. Use in accordance with claim 1, characterized by the case, that the inflammatory and hyperproliferative dermatological illnesses, respectively cutaneous manifestations of illnesses which are immunologic in origin are psoriasis, atopic dermatitis, contact dermatitis and other eczematous dermatitises, seborrhoeic dermatitis, neurodermatitis, decubitus, lichen planus, pemphigus, bullate pemphigoid, epidermolysis bullosa, urticaria, angioedemas, vasculitides, erythema, cutaneous, eosinophilias, lupus erythematosus.

3. Use in accordance with claim 1 and/or 2 characterized by use of standard carrier and/or dilution substances.

## Revendications

1. Utilisation des composés avec la formule 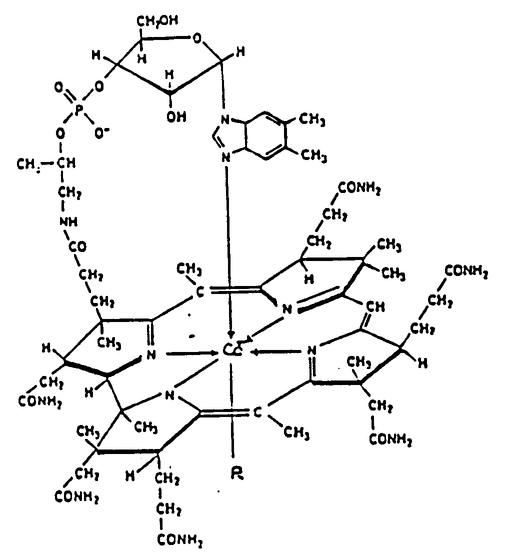 dans laquelle
R représente CN, OH, CH₃ ou H₂O,
en combinaison avec une huile ou und graisse synthétiques ou végétales pour la production d'un médicament pour le traitement topique des affections respectivement des manifestations cutanées inflammables et hyperprolifératives des maladies provoquées immunologiquement.

2. Utilisation selon revendication 1, charactérisée en ce qu'il s'agit quant aux affections respectivement manifestations cutanées inflammables et hyperprolifératives des maladies provoquées immunologiquement du psoriasis, de la dermatite atopique, de la dermatite de contact et d'autres dermatites eczémateuses, de la dermatite séborrhéique, de la neurodermatite, du décubitus, du lichen planus, du pemphigus, du pemphigoïde bulleux, de l'épidermolysis bulleuse, de l'urticaire, des angiooedèmes, des angéites, des érythèmes, des éosinophilies cutanées et du lupus érythémateux.

3. Utilisation selon revendication 1 ou 2, charactérisée en ce que des substances porteuses et/ou des agents de dilution usuels sont utilisés.
